# EUROPÄISCHE PATENTSCHRIFT

(11) **EP 2 614 851 B1**
(45) Veröffentlichungstag und Bekanntmachung des Hinweises auf die Patenterteilung: **07.08.2019**
(21) Anmeldenummer: 12151259.4
(22) Anmeldetag: 16.01.2012
(51) Int. Cl.: A61M 25/04, A61M 25/00

(54) **Katheter**
Catheter
Cathéter

(43) Veröffentlichungstag der Anmeldung: 17.07.2013
(73) Patentinhaber: Tchirikov, Michael, 06126 Halle / S. (DE); "National Laboratory Astana" Private Institution, 010000 Astana (KZ)
(72) Erfinder: Tchirikov, Michael, 06126 Halle / S. (DE)
(74) Vertreter: Gulde & Partner

(56) Entgegenhaltungen:
- US-A- 4 474 569
- US-A- 4 987 079
- US-A1- 2005 187 578

## Beschreibung

Die vorliegende Erfindung betrifft einen Katheter zur Realisierung eines Volumenstromes in ein oder aus einem menschlichen oder tierischen Organ.

Eine intrauterine Wachstumsrestriktion des Feten (Intrauterine Growth Restriction (IUGR)) kommt bei einer Plazentainsuffizienz in ungefähr in 3 bis 5 % aller Schwangerschaften vor und stellt eine schwerwiegende Komplikation während der Schwangerschaft dar. Die Wachstumsretardierung erhöht signifikant das Risiko einer Frühgeburt und ist für das schlechte neonatale Outcome, ggf. Tod des Kindes verantwortlich.

Die Sterberate bei IUGR ist um das 3- bis 10-fache erhöht. Etwa 25 bis 40 % aller intrauterin absterbenden Föten haben IUGR.

Die intrauterine Wachstumsretardation wird auch als Ursache für die Entwicklung von metabolischem Syndrom sowie für ein erhöhtes Risiko für die Entwicklung von koronaren Herzerkrankungen, Infarkten und Diabetes mellitus Typ 2 angesehen.

Bei der IUGR ist der Aminosäurentransport durch die Plazenta stark reduziert.

Es gibt keine nachgewiesene kausale Methode für die Behandlung der Plazentainsuffizienz, da eine "insuffiziente" Plazenta in der Frühschwangerschaft mit der folgenden fetalen Wachstumsretardierung am Ende des II. Trimenons entsteht.

Herkömmliche Katheter sind zur Nährstoffversorgung nicht optimal ausgestaltet, da die Nährstoffversorgung über relativ lange Zeiträume erfolgen muss und demzufolge die Gefahr besteht, dass durch Bewegungen des Fötus und/oder der Mutter der Katheter verschoben wird. US 2005/187578A1, US 4474569A, US 4987079A offenbaren Katheter mit Fixierungseinrichtungen.

Der Erfindung liegt daher die Aufgabe zu Grunde, den Transport von Nährstoffen, wie z.B. Aminosäure und Glukose, in flexibler, zuverlässiger sowie verträglicher Weise zu gewährleisten und die Schwangerschaft dadurch zu prolongieren.

Zur Lösung der Aufgabe wird erfindungsgemäß ein Katheter zur Realisierung eines Volumenstroms in ein oder aus einem menschlichen oder tierischem Organ nach Anspruch 1 zur Verfügung gestellt, der einen Katheterschlauch sowie eine zum distalen Ende des Katheters beabstandete Fixierungseinrichtung zur mechanischen Fixierung des Katheters im Gewebe eines menschlichen oder tierischen Organs aufweist. Die Fixierungseinrichtung ist insbesondere dazu eingerichtet, den Katheterschlauch in einer Plazenta oder im Cavum uteri der Gebärmutter (Amnioin) zu fixieren, um eine Positionierung des distalen Endes des Katheterschlauches z. B. in der Nabelvene (vena umbilicalis) eines Fötus zu gewährleisten.

Weitere bevorzugte Ausführungsformen können den abhängigen Ansprüchen entnommen werden. Ein Katheter kann derart ausgestaltet sein, dass die Fixierungseinrichtung bei einer am Katheterschlauch wirkenden bestimmten Zugkraft von 20 N ihre Fixierungswirkung aufgibt. Das heißt, dass der Katheter derart eingerichtet ist, dass bereits eine geringe, in Richtung des proximalen Endes gerichtete Zugkraft ausreicht, um den Katheterschlauch aus dem Gewebe herausziehen zu können. Dabei ist die bestimme Zugkraft derart bemessen, dass in Bezug zu der Fläche, auf die durchschnittlich die fixierenden Elemente der Fixierungseinrichtung auf das Gewebe wirken, die Zugfestigkeit des Gewebes ausreichend groß ist, dass bei Aufbringung der bestimmten Zugkraft eher die Fixierungseinrichtung ihre Fixierungswirkung aufgibt als dass das Gewebe beschädigt wird. Die Fixierungseinrichtung verbleibt dabei im Gewebe.

Insbesondere kann vorgesehen sein, dass die Fixierungseinrichtung bei einer am Katheterschlauch wirkenden Zugkraft von 10 N ihre Fixierungswirkung aufgibt. Vorzugsweise sollte die Fixierungswirkung bereits ab einer Zugkraft von 5 N bis 10 N aufgegeben werden, insbesondere bei Verankerung in der Plazenta. Das heißt, dass in Abhängigkeit vom jeweiligen Einsatzzweck, wie z. B. der Infusion in die vena umbilicalis und der Fixierung des Katheters in der Plazenta eine geringere Zugkraft wie z. B. von 5 oder 10 N ausreichend sein sollte, um die Fixierungswirkung aufzuheben. Bei Anwendung des Katheters zur Aminofusion nach vorzeitigem Blasensprung und einer Fixierung des Katheterschlauches an der Gebärmutterwand sollte die Fixierungseinrichtung ihre Fixierungswirkung erst bei einer Zugkraft von ca. 20 N, oder ab einer Zugkraft von 30 N aufgeben. Bei Anwendung des erfindungsgemäßen Katheters im fortgeschrittenen Schwangerschaftsstadium kann der Katheter auch derart eingerichtet sein, dass er insbesondere bei Verankerung in der Gebärmutter und dementsprechender konstruktiver Ausgestaltung die Fixierungswirkung ab einer Zugkraft von 40 N oder sogar 50 N aufgibt.

Vorteilhafterweise sollte dabei die Fixierungseinrichtung in einem Abstand von 1/10 - 1/3 der Länge des Katheters zum distalen Ende des Katheters angeordnet sein.

Dabei kann bei einem nicht erfindungsgemäßen Beispiel die Fixierungseinrichtung als ein Stent ausgebildet sein, der einen aus Maschenmaterial ausgebildeten Hohlzylinderabschnitt umfasst. Dieser Hohlzylinderabschnitt kann gegebenenfalls ein- und/oder beidseitig mit einem Hohlkegelstumpf oder einem Hohlkugelsegment versehen sein, wobei der Hohlkegelstumpf bzw. das Hohlkugelsegement vorzugsweise aus dem gleichen Maschenmaterial gefertigt ist wie der Hohlzylinderabschnitt.

Insbesondere in dieser Ausgestaltung ist der Katheter dann vorteilhaft ausgebildet, wenn der Stent sich aufgrund seiner Elastizität bei Reduzierung einer in der Längsachse des Hohlzylinders wirkenden Zugspannung aufweitet. Die Reduzierung einer auf den Hohlzylinder wirkenden Zugspannung tritt z. B. dann ein, wenn ein Mandrain aus dem Katheterschlauch herausgezogen wird, wodurch eine durch den Mandrain auf den Katheterschlauch zuvor aufgebrachte Zugspannung aufgehoben wird. Dabei weitet sich der Stent aufgrund seiner Elastizität auf, sodass sich sein Durchmesser vergrößert und er sich an die Wandung einer Öffnung im Gewebe kraft- und/oder formschlüssig anlegen kann und somit eine relativ feste mechanische Verbindung zwischen dem Gewebe und dem Katheterschlauch herstellen kann.

Hinzukommend oder in alternativer Ausführungsform des Stents kann dieser derart ausgestaltet sein, dass er sich bei Wärmezufuhr ausweitet. Das heißt, dass er bei Erhöhung seiner Temperatur derart seine Form ändert, dass sich sein Durchmesser vergrößert, sodass er sich an die Wandung einer Öffnung im Gewebe kraft- und/oder formschlüssig anlegen kann und derart eine relativ feste Verbindung zwischen dem Gewebe und dem Katheterschlauch herstellen kann. Diese Eigenschaft des Stents kann z. B. durch eine geeignete Formgedächtnislegierung als Maschenmaterial realisiert sein.

In der erfindungsgemäßen Ausführungsform ist vorgesehen, dass die Fixierungseinrichtung ein Spreizelement ist, welches im Querschnitt vergleichsweise einen größeren Abstand zur Längsache des Katheterschlauches aufweist als die Außenseite des Katheterschlauches. Mit dem diesbezüglichen Querschnitt ist der quer zur Längsachse des Katheterschlauches verlaufende Querschnitt gemeint. Durch diese Ausbildung der Fixierungseinrichtung als Spreizelement wird eine Art Widerhaken ausgebildet, der eine mechanische Fixierung des Katheterschlauches im Gewebe des Organs ermöglicht.

Es kann dabei insbesondere vorgesehen sein, dass das Spreizelement elastisch ausgebildet ist und der Katheterschlauch im Wesentlichen tangential zum Spreizelement verläuft. In besonders bevorzugter Ausführungsform verläuft der Katheterschlauch genau tangential zum Spreizelement. Das Elastizitätsmodul des Spreizelementes sollte dabei 0,01 bis 0,3 kN/mm² betragen. Das Spreizelement kann ein schräg geschnittenes Rohrsegment sein, welches auf den Katheterschlauch aufgeschoben ist, oder es kann ein integraler Abschnitt des Katheterschlauches sein.

In einer Ausführungsalternative ist vorgesehen, dass der Katheter zwischen der Fixierungseinrichtung und dem Katheterschlauch eine kraft-, form- und/oder stoffschlüssige Verbindung aufweist, und diese kraft-, form- und/oder stoffschlüssige Verbindung derart ausgeführt ist, dass sie sich bei der am Katheterschlauch wirkenden bestimmten Zugkraft löst. Dabei kann die Verbindung zwischen dem Katheterschlauch und der Fixierungseinrichtung durch eine integrale Ausführung des Katheterschlauches ausgebildet sein, sodass das Katheterschlauch-Material einen Vorsprung bzw. eine Auskragung als Spreizelement ausbildet. In diesem Fall ist der die Fixierungseinrichtung mit dem Katheterschlauch verbindende Materialabschnitt derart ausgelegt, dass er ab der bestimmten, am Katheterschlauch wirkenden Zugkraft abschert, reißt oder umbiegt, sodass keine Fixierungswirkung mehr aufrecht erhalten bleibt.

In einer beispielhaften Ausführungsform ist die Fixierungseinrichtung mittels einer Klebeverbindung, das heißt stoffschlüssig, mit dem Katheterschlauch verbunden, oder wie eine Manschette kraft- und/oder formschlüssig mit dem Katheterschlauch verbunden.

In diesen Ausgestaltungen ist die Klebeverbindung bzw. die Manschette mit ihrer radialen Spannkraft und/oder formschlüssig realisierten Haltekraft derart ausgeführt, dass sie sich ab der bestimmten, am Katheterschlauch wirkenden Zugbelastung löst. Dabei ist auch eine Kombination der Ausführungsform mit Klebeverbindung und Manschette nicht ausgeschlossen.

Eine weitere Ausführungsalternative ist es, dass der Katheter im Bezug zur Position der Fixierungseinrichtung distal ein Formelement auf oder am Katheterschlauch aufweist, dessen größte Ausdehnung im Querschnitt größer ist als der Querschnitt des Katheterschlauches, wobei der Katheter zwischen dem Formelement und dem Katheterschlauch eine kraft-, form- und/oder stoffschlüssige Verbindung aufweist und diese kraft-, form- und/oder stoffschlüssige Verbindung derart ausgeführt ist, dass sie sich bei der bestimmten, am Katheterschlauch wirkenden Zugkraft löst. Die größte Ausdehnung des Formelementes ist somit auch größer als die lichte Weite der Öffnung in der Fixierungseinrichtung, durch die der Katheterschlauch geführt ist. Das Formelement verhindert im normalen Gebrauch eine Verschiebung der Fixierungseinrichtung in Bezug zum Katheterschlauch. Wird jedoch die bestimmte Zugkraft auf das proximale Ende des Katheterschlauches aufgebracht, löst sich die Verbindung zwischen dem Formelement und dem Katheterschlauch, sodass der Katheterschlauch aus dem Gewebe herausgezogen werden kann und die Fixierungseinrichtung im Gewebe verbleibt. Das genannte Formelement fungiert hierbei sozusagen als Blockierungselement zur Blockierung einer Relativbewegung zwischen der Fixierungseinrichtung und dem Katheterschlauch bei normaler Nutzung des Katheters. Dabei muss das Formelement nicht unbedingt mit der Fixierungseinrichtung fest verbunden sein.

Auch hier kann das Formelement mittels einer Klebeverbindung, das heißt stoffschlüssig, mit dem Katheterschlauch verbunden sein, oder kraft- und/oder formschlüssig mit dem Katheterschlauch verbunden sein. In diesen Ausgestaltungen ist die Klebeverbindung bzw. die radiale Spannkraft und/oder die formschlüssig realisierte Haltekraft derart ausgeführt, dass sie sich ab der bestimmten, am Katheterschlauch wirkenden Zugbelastung löst.

In einer weiteren Ausführungsalternative weist der Katheter im Bezug zur Position der Fixierungseinrichtung ein distales Formelement auf oder am Katheterschlauch auf, dessen größte Ausdehnung im Querschnitt größer ist als der Querschnitt des Katheterschlauches, wobei die Fixierungseinrichtung derart ausgestaltet ist, dass sie sich aufgrund der bestimmten am Katheterschlauch wirkenden Zugkraft an ihrem dem Katheterschlauch sowie dem Formelement zugewandten Rand derart aufweitet, dass der Katheterschlauch zusammen mit dem Formelement durch die Fixierungseinrichtung gezogen werden kann. Das Formelement wandert dabei von der distalen Seite der Fixierungseinrichtung zur proximalen Seite der Fixierungseinrichtung. Diese Ausgestaltung bietet sich insbesondere dann an, wenn die Fixierungseinrichtung den aus Maschenmaterial ausgebildeten Hohlzylinderabschnitt umfasst, an dem zumindest an der Seite des Formelementes ein Hohlkegelstumpf oder ein Hohlkugelsegment angeordnet ist. Diese weiten sich erst bei Aufbringung der bestimmten Zugkraft am Katheterschlauch so weit auf, dass das Formelement durch die Fixierungseinrichtung hindurchgezogen werden kann.

In weiterer, alternativer Ausführungsform ist vorgesehen, dass das Spreizelement eine derartige Biegesteifigkeit aufweist, dass es bei Wirkung einer Zugkraft am Katheterschlauch seine Ausrichtung von der proximalen Seite zur distalen Seite ändert. Das heißt, dass bevorzugt vorgesehen ist, dass zumindest eine Richtungskomponente der Längsachse des Spreizelementes bei Wirkung der bestimmten Zugkraft umgekehrt wird, nämlich von der proximalen Seite zur distalen Seite.

Dabei kann die Zugkraft, ab der die Richtungsänderung des Spreizelementes bewirkt wird, die bestimmte Zugkraft sein, wobei in dieser Ausgestaltung der Katheter vorzugsweise nicht derart ausgeführt ist, dass sich eine Verbindung zwischen der Fixierungseinrichtung und dem Katheterschlauch bei Einwirkung der bestimmten Zugkraft löst, sondern dass die Fixierungswirkung durch ein Umkippen bzw. Umschwenken des Spreizelementes aufgegeben wird.

Es kann der Katheter auch derart ausgestaltet sein, dass die Zugkraft, ab der die Richtungsänderung des Spreizelementes bewirkt wird, geringer ist als die bestimmte Zugkraft, sodass bei einer geringeren Zugkraft als der bestimmten Zugkraft zunächst eine Richtungsänderung des Spreizelementes zu verzeichnen ist, die Fixierungswirkung durch das Spreizelement jedoch noch aufrecht erhalten bleibt. Steigt die Zugkraft bis auf den Wert der bestimmten Zugkraft an, kommt es zu der beschriebenen Lösung der Fixierungseinrichtung vom Katheterschlauch in dem Sinne, dass eine Relativbewegung zwischen der Fixierungseinrichtung und dem Katheterschlauch ermöglicht wird.

In weiterer, vorteilhafter Ausgestaltung ist vorgesehen, dass der erfindungsgemäße Katheter an seinem proximalen Ende ein Port-System aufweist. Unter diesem Port-System ist im Sinne der Erfindung ein subkutan positionierbarer Hohlraum zu verstehen, der an den Katheterschlauch angeschlossen ist und zur Aufnahme von Wirkstoffen wie z. B. Aminosäuren und Glukose sowie zur Infusion dieser Wirkstoffe durch den Katheterschlauch eingerichtet ist.

Die Erfindung wird im Folgenden anhand der in den beiliegenden Zeichnungen dargestellten Ausführungsbeispiele erläutert.

Es zeigen
Figur 1 einen nicht erfindungsgemäßen Katheter mit Stent in perspektivischer Ansicht,
Figur 2 die Seitenansicht eines Abschnittes eines erfindungsgemäßen Katheters mit zwei Spreizelementen, und
Figur 3 die Seitenansicht eines Abschnittes eines erfindungsgemäßen Katheters mit einem Spreizelement.

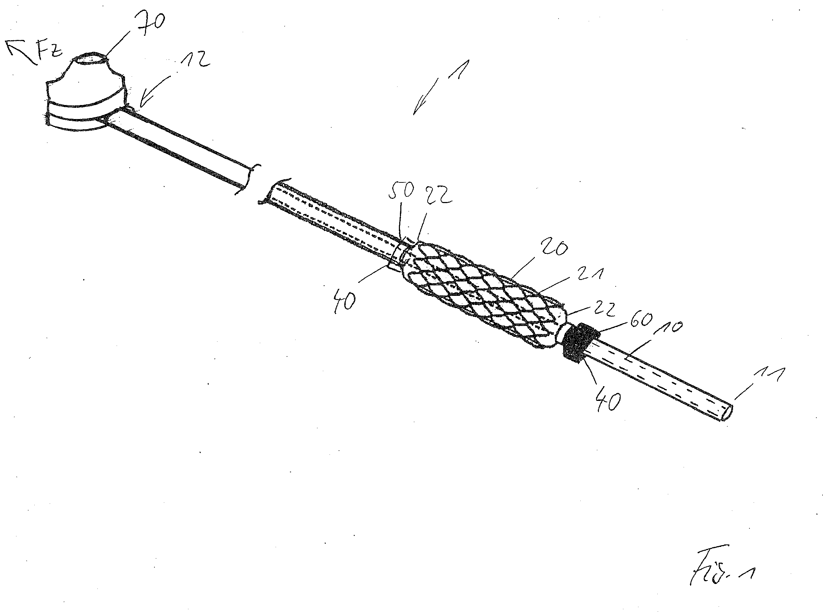

Insbesondere aus Figur 1 ist der Gesamt-Aufbau eines nicht erfindungsgemäßen Katheters 1 ersichtlich. Es handelt sich hierbei um einen Katheter 1, der durch die Bauchdecke und Vorderwandplazenta der Mutter direkt in die Nabelschnurvene des Kindes unter Ultraschallkontrolle eingeführt werden kann. Das proximale Ende 12 des Katheters 1 weist ein Port-System 70 auf. Das Port-System 70 ist subkutan implantierbar. Durch dieses Port-System 70 kann dem Fötus direkt intravenös Aminosäuren und Glukose kontinuierlich infundiert werden.

Dabei ist der nicht erfindungsgemäße Katheter 1 nicht maßstäblich dargestellt, sondern ist für gewöhnlich wesentlich länger als in Figur 1 gezeigt. Die Ausführungsform des nicht erfindungsgemäßen Katheters 1 in Figur 1 weist einen Stent mit einem Hohlzylinderabschnitt 21 aus einem Maschenmaterial auf, der beidseitig einen Hohlkegelstumpf bzw. ein Hohlkugelsegment 22 mit geeigneter Öffnung zum Durchlass des Katheterschlauches 10 aufweist. In Richtung des distalen Endes 11 des Katheterschlauches 10 weist der Katheter 1 ein Formelement 60 auf, dessen Durchmesser größer ist als der des Katheterschlauches 10. Proximal zum Hohlzylinderabschnitt 21 ist eine Manschette 50 angeordnet, welche fest mit dem Hohlzylinderabschnitt 21, ggf. über einen Hohlkegelstumpf 22 oder ein Hohlkugelsegment 22, verbunden ist.

Zwischen der Manschette 50 und dem Katheterschlauch 10 sowie auch zwischen dem Formelement 60 und dem Katheterschlauch 10 besteht eine kraft-, form- und/oder stoffschlüssige Verbindung 40.

Dabei ist der Katheter 1 nicht auf die vorliegende Ausgestaltung mit dem distal angeordneten Formelement 60 und der proximal angeordneten Manschette 50 eingeschränkt, sondern das Formelement 60 sowie die Manschette 50 sind in Figur 1 lediglich zur Verdeutlichung mehrerer unterschiedlicher Aspekte der Erfindung am Katheterschlauch 10 angeordnet. In bestimmten Ausführungsvarianten des Katheters 1 kann dieser mit dem Formelement 60, aber ohne Manschette 50, oder umgekehrt mit der Manschette 50 aber ohne das Formelement 60 ausgestaltet sein.

Bei Implantation des Katheters 1 wird ein Mandrain durch das proximale Ende 12 des Katheters 1 eingeschoben, sodass die bei der Einführung des Mandrains auf den Katheterschlauch 10 wirkenden Reibkräfte diesen dehnen, und der Hohlzylinderabschnitt 21 einer Zugbelastung ausgesetzt wird. Aufgrund dieser Zugbelastung verlängert sich der Hohlzylinderabschnitt 21 und verringert dabei seinen Durchmesser. In dieser Form lässt sich der Katheter 1 und insbesondere der Hohlzylinderabschnitt 21 optimal in einer Öffnung im Gewebe platzieren. Wird der Mandrain wieder aus dem proximalen Ende 12 des Katheters 1 herausgezogen, verringert sich die Zugspannung im Katheterschlauch 10 und somit auch im Hohlzylinderabschnitt 21, sodass sich der Hohlzylinder 21 wieder zusammenzieht und dabei seinen Durchmesser vergrößert. Die elastischen Rückstellkräfte bewirken dabei Andruckkräfte an die Wandung der Gewebeöffnung, sodass der Hohlzylinderabschnitt 21 und, bedingt durch die mechanische Verbindung über die Manschette 50 und/oder über das Formelement 60, auch der Katheterschlauch 10 im Gewebe fixiert ist. Dadurch wird erreicht, dass das distale Ende 11 im Wesentlichen zuverlässig in der gewünschten Position verbleibt, auch wenn der Fötus oder die Mutter Bewegungen ausführen. Durch das Port-System können Nährstoffe dosiert werden und durch den Katheterschlauch 10 infundiert werden.

Der Katheter 1 kann dabei derart ausgeführt sein, dass die zwischen der Manschette 50 und dem Katheterschlauch 10 kraft-, form- und/oder stoffschlüssige Verbindung 40 sich dann löst, wenn am proximalen Ende 12 eine bestimmte Zugkraft Fz, von z. B. 10 N eingeleitet wird. Die Folge davon ist, dass der Hohlzylinderabschnitt 21 im Gewebe verbleibt und der Katheterschlauch 10 aus dem Hohlzylinderabschnitt 21 herausgezogen werden kann. In dieser Ausgestaltung sollte auf die dargestellte Anordnung des Formelementes 60 vorzugsweise verzichtet werden.

Die Manschette kann auch auf der distalen Seite des Hohlzylinderabschnitts 21, also an Stelle des Formelements 60, angeordnet sein. Ist der Katheter 1 jedoch mit dem Formelement 60 ausgestaltet, sollte der Hohlzylinderabschnitt 21 und/oder der distal am Hohlzylinderabschnitt 21 angeordnete Hohlkegelstumpf bzw. ein dort angeordnetes Hohlkugelsegment 22 derart ausgestaltet sein, dass bei der bestimmten Zugkraft Fz von z. B. 10 N am proximalen Ende 12 der jeweilige Hohlkegelstumpf bzw. das jeweilige Hohlkugelsegment 22 und/oder der Hohlzylinderabschnitt 21 derart aufgeweitet werden, dass das Formelement 60 durch diese hindurch gleiten kann, ohne dass dabei eine Relativbewegung zwischen dem Formelement 60 und dem Katheterschlauch 10 stattfindet. In dieser Ausführungsform sollte am proximalen Ende des Hohlzylinderabschnittes 21 vorzugsweise eine Manschette 50 angeordnet sein.

Das Beispiel ist jedoch nicht darauf eingeschränkt, den Katheter 1 entweder mit dem Formelement 60 oder Manschette 50 auszuführen, sondern es können an einem Katheter sowohl die Manschette 50 als auch das Formelement 60 gleichzeitig angeordnet sein, und ihre jeweilige Wirkung entfalten, wobei sie in diesem Falle derart auszugestalten sind, dass die Manschette 50 sowie auch das Formelement 60 den Hohlzylinderabschnitt 21 mit derartigen Kräften am Katheterschlauch fixieren, dass die Fixierung trotzdem ab der bestimmten Zugkraft Fz am proximalen Ende aufgegeben wird.

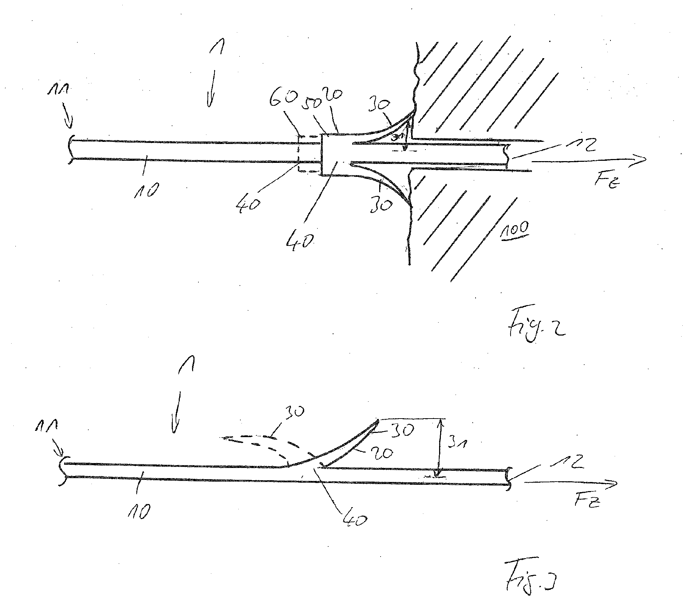

In den Figuren 2 und 3 sind erfindungsgemäße Ausführungsformen des Katheters 1 dargestellt, wobei die Fixierungseinrichtung 20 in Figur 2 durch 2 Spreizelemente 30 realisiert ist, die mittels einer kraft-, stoff- und/oder formschlüssigen Verbindung mit dem Katheterschlauch 10 verbunden sind. Die in Richtung des proximalen Endes 12 des Katheterschlauches 10 weisenden Enden der Spreizelemente 30 weisen einen größeren Abstand zur Längsachse 31 des Katheterschlauches 10 auf als dessen Oberfläche. Dadurch ist gewährleistet, dass der Katheterschlauch 10 bei Anlage der Spreizelemente 30 am Gewebe 100 zumindest nicht in Richtung des proximalen Endes 12 verschoben werden kann. Die Spreizelemente 30 sind dabei bevorzugt aus einem elastischen Material wie z. B einem relativ weichen Kunststoff gefertigt, sodass sie durch die Öffnung im Gewebe 100 hindurch geführt werden können und sich nach Hindurchführung aufweiten können, sodass sie, wie in Figur 2 dargestellt, am Gewebe 100 anliegen können.

In ähnlicher Ausführungsform wie in Figur 1 angedeutet kann auch bei diesem Katheter 1 mit Spreizelementen 30 ein Formelement 60 auf der distalen Seite der Fixierungseinrichtung 20 angeordnet sein, wobei in diesem Falle die kraft-, form- und/oder stoffschlüssige Verbindung 40 vorzugsweise zwischen dem Formelement 60 und dem Katheterschlauch 10 hergestellt ist. Diese Verbindung 40, die auch in der Fixierungseinrichtung 20 selbst und/oder im Formelement 60 vorhanden sein kann, ist derart ausgeführt, dass sie sich bei Einwirkung der bestimmten Zugkraft Fz am proximalen Ende 12 löst. Dadurch kann der Katheterschlauch 10 aus der Fixierungseinrichtung 20 gezogen werden.

Alternativ kann die Fixierungseinrichtung 20 derart ausgestaltet sein, dass sie sich bei Einwirkung der bestimmten Zugkraft Fz am proximalen Ende 12 derart aufweitet, dass das weiterhin fest auf dem Katheterschlauch 10 angeordnete Formelement 60 sich durch die Fixierungseinrichtung 20 bewegt und derart die Fixierungswirkung aufgegeben wird.

Eine weitere Ausführungsalternative ist in Figur 3 dargestellt, bei der die Fixierungseinrichtung 20 aus einem Spreizelement 30 besteht, welches ein integraler Bestandteil des Materials des Katheterschlauches 10 sein kann. Dabei ist die vorliegende Erfindung nicht auf die in den Figuren 2 und 3 gezeigte Anzahl der Spreizelemente eingeschränkt, sondern es kann in jeder der gezeigten Ausführungsformen eine unterschiedliche Anzahl von Spreizelementen 30 angeordnet sein.

Das in Figur 3 dargestellte Spreizelement ist derart ausgeführt, dass es bei der bestimmten Zugkraft Fz am proximalen Ende 12 des Katheterschlauches 10 seine Ausrichtung vom proximalen Ende 12 in Richtung des distalen Endes 11 ändert, und sich bevorzugt derart dicht an den Katheterschlauch 10 anlegt, dass der Katheterschlauch mit dem Spreizelement 30 durch eine Öffnung im Gewebe gezogen werden kann. Auch in dieser Ausgestaltung ist der Abstand 31 von der Spitze des Spreizelementes 30 zur Längsachse des Katheterschlauches 10 derart ausgeführt, dass eine Verschiebung des Katheterschlauches 10, so lange nicht die bestimmte Zugkraft Fz aufgebracht wird, verhindert wird.

Mit den dargestellten Ausführungen des erfindungsgemäßen Katheters kann dessen Katheterschlauch in einfacher, zuverlässiger sowie flexibler und verträglicher Weise in der Plazenta oder im Cavum uteri platziert werden.

Um die Entfernung des Katheters 1 bei bestimmten Indikationen zu gewährleisten, z.B. bei einem vorzeitigen Blasensprung, wird in den Katheter 1 ein Mandrain eingeführt, wodurch der Katheter 1 und die Fixierungseinrichtung 20 auseinander gezogen werden. Die Fixierungseinrichtung 20 verringert dabei ihren Durchmesser, so dass sie zusammen mit dem Katheterschlauch 10 herausgezogen werden kann.

Falls jedoch eine sehr kurzfristige Entfernung des Katheters 1 notwendig ist oder die Einführung des Mandrains nicht praktikabel ist, lässt sich die Fixierungswirkung der Fixierungseinrichtung 20 einfach durch die Aufbringung der bestimmten Zugkraft am proximalen Ende 12 aufheben, so dass der Katheterschlauch 10 freigegeben wird und heraus ziehbar ist. Die Fixierungseinrichtung 20 verbleibt in der Plazenta bzw. in der Gebärmutter und wird mit der Plazenta und den Eihäuten später geboren.

Über den Katheter 1 kann eine Infusion von Aminosäuren und Glukose zur Behandlung der IUGR appliziert werden.

Damit kann eine direkte intravenöse intrauterine Gabe von Medikamenten (z.B. Digoxin und Amiodaron bei fetaler Tachykardie) gewährleistet werden.

Der Katheter 1 kann allerdings auch für andere Infusionen, z.B. für die kontinuierliche Amnioinfusion nach dem vorzeitigen Blasensprung oder auch für die Ableitung der Flüssigkeit aus Organen oder körperlichen Hohlräumen bei Menschen und Tieren verwendet werden. Der Katheter 1 ist besonders gut für die intrauterine Anwendung bei erkrankten Feten mit einer infra-/supravesikalen Obstruktion, Hydrothorax, Zysten usw. geeignet.

**Bezugszeichenliste**

| | |
|---|---|
| Katheter | 1 |
| | |
| Katheterschlauch | 10 |
| distales Ende | 11 |
| proximales Ende | 12 |
| | |
| Fixierungseinrichtung | 20 |
| Hohlzylinderabschnitt | 21 |
| Hohlkegelstumpf, Hohlkugelsegment | 22 |
| | |
| Spreizelement | 30 |
| Abstand zur Längsachse | 31 |
| | |
| Verbindung | 40 |
| | |
| Manschette | 50 |
| | |
| Formelement | 60 |
| | |
| Port-System | 70 |
| | |
| Gewebe | 100 |
| | |
| Zugkraft | Fz |

## Patentansprüche

1. Katheter (1) zur Realisierung eines Volumenstromes in ein oder aus einem menschlichen oder tierischen Organ, umfassend einen Katheterschlauch (10) sowie eine zum distalen Ende (11) des Katheters (1) beabstandete Fixierungseinrichtung (20) zur mechanischen Fixierung des Katheters (1) im Gewebe (100) eines menschlichen oder tierischen Organs,
wobei die Fixierungseinrichtung (20) wenigstens ein Spreizelement (30) umfasst, das wenigstens bereichsweise in Richtung eines proximalen Endes (12) des Katheterschlauchs (10) einen im Querschnitt größeren Abstand zur Längsachse (31) des Katheterschlauchs (10) aufweist, als eine Außenseite des Katheterschlauchs (10), **dadurch gekennzeichnet, dass** das Spreizelement (30) ausgebildet ist, bei Wirksamkeit einer definierten Zugkraft (Fz) von 5 N bis 10 N am Katheterschlauch (10), seine Ausrichtung vom proximalen Ende (12) in Richtung des distalen Endes (11) zu ändern.

2. Katheter nach Anspruch 1, **dadurch gekennzeichnet, dass** das Spreizelement (30) ausgebildet ist, bei Wirksamkeit einer definierten Zugkraft (Fz) von 10 N am Katheterschlauch (10), seine Ausrichtung vom proximalen Ende (12) in Richtung des distalen Endes (11) zu ändern.

3. Katheter nach Anspruch 1 oder 2, **dadurch gekennzeichnet, dass** eine Änderung der Ausrichtung des Spreizelements (30) vom proximalen Ende (12) in Richtung des distalen Endes (11) bei Wirksamkeit der definierten Zugkraft (Fz) dadurch erreicht wird, dass das Spreizelements (30) eine entsprechende Biegesteifigkeit aufweist.

4. Katheter nach einem der vorhergehenden Ansprüche, **dadurch gekennzeichnet, dass** der Katheterschlauch (10) im Wesentlichen tangential zum Spreizelement (30) verläuft.

5. Katheter nach einem der vorhergehenden Ansprüche, **dadurch gekennzeichnet, dass** die Fixierungseinrichtung (20) mehrere Spreizelemente (30) umfasst.

6. Katheter nach einem der vorhergehenden Ansprüche, **dadurch gekennzeichnet, dass** die Fixierungseinrichtung (20) in einem Abstand von 1/10 bis 1/3 der Länge des Katheters (1) zu seinem distalen Ende (11) angeordnet ist.

7. Katheter nach einem der vorhergehenden Ansprüche, **dadurch gekennzeichnet, dass** der Katheter (1) zwischen der Fixierungseinrichtung (20) und dem Katheterschlauch (10) eine kraft-, form- und/oder stoffschlüssige Verbindung (40) aufweist.

8. Katheter nach einem der vorhergehenden Ansprüche, **dadurch gekennzeichnet, dass** der Katheter an seinem proximalen Ende (12) ein Port-System (70) aufweist.

## Claims

1. A catheter (1) for implementing a volumetric flow into or out of a human or animal organ, comprising a catheter tube (10) and a fixation device (20), being distanced from the distal end (11) of the catheter (1), for mechanically fixing the catheter (1) within the tissue (100) of a human or animal organ,
wherein
the fixation device (20) comprises at least one spreading element (30) that, in the direction of a proximal end (12) of the catheter tube (10), at least partially has, in cross-section, a larger distance to the longitudinal axis (31) of the catheter tube (10) than an external side of the catheter tube (10),
**characterized in that** the spreading element (30) is designed to change its orientation from the proximal end (12) in the direction of the distal end (11) when a defined tensile force (Fz) of 5 N to 10 N acts on the catheter tube (10).

2. The catheter according to Claim 1, **characterized in that** the spreading element (30) is designed to change its orientation from the proximal end (12) in the direction of the distal end (11) when a defined tensile force (Fz) of 10 N acts on the catheter tube (10).

3. The catheter according to Claim 1 or 2, **characterized in that** a change in the orientation of the spreading element (30) from the proximal end (12) in the direction of the distal end (11) when the defined tensile force (Fz) is active is achieved by the spreading element (30) having an appropriate flexural strength.

4. The catheter according to any one of the preceding claims, **characterized in that** the catheter tube (10) runs substantially tangentially to the spreading element (30).

5. The catheter according to any one of the preceding claims, **characterized in that** the fixation device (20) comprises a plurality of spreading elements (30).

6. The catheter according to any one of the preceding claims, **characterized in that** the fixation device (20) is arranged at a distance from its distal end (11), said distance being 1/10 to 1/3 of the length of the catheter (1)

7. The catheter according to any one of the preceding claims, **characterized in that** the catheter (1) has a form-fit, force-fit, and/or material-fit connection (40) between the fixation device (20) and the catheter tube (10).

8. The catheter according to any one of the preceding claims, **characterized in that** the catheter has a port (70) at its proximal end (12).

## Revendications

1. Cathéter (1) destiné à réaliser un débit volumétrique entrant ou sortant d'un organe humain ou animal, comprenant un tube de cathéter (10) ainsi qu'un dispositif de fixation (20) espacé de l'extrémité distale (11) du cathéter (1) pour la fixation mécanique du cathéter (1) dans le tissu (100) d'un organe humain ou animal,
le dispositif de fixation (20) comprenant au moins un élément d'écartement (30), qui présente, au moins en partie, dans la direction d'une extrémité proximale (12) du tube de cathéter (10), une distance en coupe transversale par rapport à l'axe longitudinal (31) du tube de cathéter supérieure à celle d'un côté extérieur du tube de cathéter (10),
**caractérisé en ce que** l'élément d'écartement (30) est conçu pour modifier son orientation de l'extrémité proximale (12) vers l'extrémité distale (11) sous l'effet d'une force de traction (Fz) définie de 5 N à 10 N sur le tube de cathéter (10).

2. Cathéter selon la revendication 1, **caractérisé en ce que** l'élément d'écartement (30) est conçu pour modifier son orientation de l'extrémité proximale (12) vers l'extrémité distale (11) sous l'effet d'une force de traction (Fz) définie de 10 N sur le tube de cathéter (10).

3. Cathéter selon la revendication 1 ou 2, **caractérisé en ce qu'**une modification de l'orientation de l'élément d'écartement (30) de l'extrémité proximale (12) vers l'extrémité distale (11) sous l'effet de la force de traction (Fz) définie est obtenue par le fait que l'élément d'écartement (30) présente une rigidité à la flexion correspondante.

4. Cathéter selon l'une quelconque des revendications précédentes, **caractérisé en ce que** le tube de cathéter (10) s'étend de manière sensiblement tangentielle à l'élément d'écartement (30).

5. Cathéter selon l'une quelconque des revendications précédentes, **caractérisé en ce que** le dispositif de fixation (20) comprend plusieurs éléments d'écartement (30).

6. Cathéter selon l'une quelconque des revendications précédentes, **caractérisé en ce que** le dispositif de fixation (20) est disposé à une distance de 1/10 à 1/3 de la longueur du cathéter (1) par rapport à l'extrémité distale (11) de celui-ci.

7. Cathéter selon l'une quelconque des revendications précédentes, **caractérisé en ce que** le cathéter (1) présente un assemblage (40) par force, par complémentarité de forme et/ou par liaison de matière entre le dispositif de fixation (20) et le tube de cathéter (10).

8. Cathéter selon l'une quelconque des revendications précédentes, **caractérisé en ce que** le cathéter présente un système d'orifice (70) sur son extrémité proximale (12).
